(19) Europäisches Patentamt
European Patent Office
Office européen des brevets

(11) **EP 2 361 604 B1**

(12) **EUROPEAN PATENT SPECIFICATION**

(45) Date of publication and mention
of the grant of the patent:
**27.08.2014 Bulletin 2014/35**

(51) Int Cl.:
*A61K 8/38* *(2006.01)*     *A61K 8/44* *(2006.01)*
*A61Q 5/10* *(2006.01)*

(21) Application number: **10001713.6**

(22) Date of filing: **19.02.2010**

(54) **Powder hair dye composition based on sodium percarbonate and storage package therefor**

Pulverförmige Haarfärbezusammensetzung  enthaltend Natriumpercarbonat und Aufbewahrungsverpackung dafür

Composition de teinture capillaire en poudre à base de percarbonate de sodium et son emballage de stockage

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**31.08.2011 Bulletin 2011/35**

(73) Proprietor: **Hoyu Co., Ltd.**
**Nagoya-shi**
**Aichi, 461-8650 (JP)**

(72) Inventors:
 • **Hasegawa, Mikinobu**
  **Aichi-gun**
  **Aichi 480-1131 (JP)**
 • **Tanaka, Motoharu**
  **Aichi-gun**
  **Aichi 480-1131 (JP)**

(74) Representative: **Kuhnen & Wacker**
**Patent- und Rechtsanwaltsbüro**
**Prinz-Ludwig-Straße 40A**
**85354 Freising (DE)**

(56) References cited:
EP-A1- 1 447 380     EP-A2- 1 213 009
WO-A2-02/051745     CN-A- 101 337 658
DE-A1- 19 533 524     FR-A1- 2 924 123
JP-A- 2002 097 120     JP-A- 2003 104 856
JP-A- 2003 104 857     JP-A- 2003 238 369

 • DATABASE WPI Week 200682 Thomson Scientific, London, GB; AN 2006-800698 XP002598845 & CN 1 807 228 A (SHANDONG ZHONGCHENG CHEM IND CO LTD) 26 July 2006 (2006-07-26)
 • DATABASE WPI Week 199936 Thomson Scientific, London, GB; AN 1999-425291 XP002598846 & JP 11 172286 A (KAO CORP) 29 June 1999 (1999-06-29)

Remarks:
The file contains technical information submitted after the application was filed and not included in this specification

## Description

Technical Field

The present invention relates to a storage package obtained by placing a powder hair dye composition for dyeing hair in a storage container as defined in claim 1.

Technical terms given for use in accordance with the invention are individually defined as follows.

The term "hair dye composition" encompasses at least oxidation hair dyes and hair bleaches.

The "oxidation hair dyes" are used for the purpose of dyeing hair, and contain at least an oxidizing agent and an oxidation dye and generally contain an alkaline agent. The oxidation dye comprises a primary intermediate or comprises a primary intermediate and a coupler. When such oxidation hair dye interacts with hair, melanin in the hair is decomposed through the action of the oxidizing agent. Concurrently, the oxidation dye is polymerized together inside the hair to form an oxidized dye polymer, so that the hair is dyed to a desired color. To control the color of dyed hair, the oxidation hair dye may sometimes be blended with a direct dye.

The "hair bleaches" are used for the purpose of hair bleaching, and contain at least an oxidizing agent and generally contain an alkaline agent as well. When such hair bleaches interact with hair, melanin in hair is decomposed through the action of the oxidizing agent, so that the hair is bleached to a bright color.

The "powder hair dye composition" is a powdery preparation of a hair dye composition, in which individual components, for example an oxidizing agent, an oxidation dye and an alkaline agent in powdery forms are blended. On use, the powder hair dye composition is dissolved in a solution for the dissolution, such as water, for hair application. In the present description, the contents of the individual components in the powder hair dye composition are represented in numerical figure in unit "% by mass" when the individual components are at powdery states before the dissolution thereof in such solution.

The "active oxygen amount" regarding sodium percarbonate is represented in numerical figure in unit "% by mass" of the amount of oxygen gas generated via the decomposition of solid (powder) sodium percarbonate to the amount of powder sodium percarbonate as a raw material. Theoretically, the amount of oxygen gas generated via the decomposition of sodium percarbonate should be at a. predetermined value. Because commercially available powder sodium percarbonate is contaminated with sodium carbonate ($Na_2CO_3$) at various ratios, however, the active oxygen amount is not at a constant value.

The "storage package" means a storage container placing therein a powder hair dye composition.

Background Art

Oxidation hair dyes and hair bleaches as hair dyes in powder are provided along with oxidation hair dyes and hair bleaches in cream and liquid. Hair dyes in cream and liquid are mostly of two-agent types. The hair dyes are used commonly in such a manner that the two agents are mixed together on use. However, powder hair dyes are generally of single-agent types, and are applied to hair after the powder hair dyes are dissolved in such solution.

[Patent Literature 1] US 5,575,989 A

Patent Literature 1 discloses "Powder Bleaching Compositions for Hair and Method of Use thereof". The composition comprises a conditioner, a bleach accelerator, a thickener, a silica component and a hydrogen peroxide source, with a sufficient amount of water to provide a water activated composition. The composition prevents damage to the hair during bleaching and provides limited ammonia odor during use.

[Patent Literature 2] JP 2002 97120 A

Patent Literature 2 discloses "a powder hair dye composition". The composition contains at least a dye, an oxidizing agent, a dispersant and an oily matter, where sodium percarbonate is disclosed as a preferable oxidizing agent. However, the literature never describes anything about "active oxygen amount" from sodium percarbonate. The reason of preference given to sodium percarbonate is the great dyeability thereof.

[Patent Literature 3] JP 2000-255632 A

Patent literature 3 discloses an invention of "a package of a peroxide-containing article". The container wall of the package is of a three-layer structure comprising (1) an oxygen permeation layer using a polyethylene resin and the like with good oxygen gas permeability, (2) an oxygen absorption layer using a resin composition in blend with a deoxygen composition of iron series, and (3) a gas barrier layer using a gas barrier material.

[Patent Literature 4] JP 2003 104856 A

Patent literature 4 discloses a "hair dye". This powdered hair dye, which presents red purple color, is prepared by blending together 2 g of p-phenylenediamine sulfate (dye component A), 2 g of 5-amino-2-methylphenol sulfate (dye component B), 50 g of sodium percarbonate, 15 g of ammonium sulfate, 3 g of sodium lauryl sulfate, 27 g of sodium carboxymethylcellulose, 1 g of disodium edetate, 1 g of a perfume, and 3 g of sodium sulfate.

[Patent Literature 5] JP 2003 104857 A

Patent literature 5 discloses a "hair die". This powdered hair dye having red color, is prepared by blending together 0.2 g of p-phenylenediamine sulfate (dye component A) and/or 0.1 g of 2-chloro-p-phenylenediamine sulfate (dye component A), 1.5 g of p- aminophenol sulfate (dye component B), 1 g of 2,4-diaminophenoxyethanol sulfate (dye component C), 50 g of sodium percarbonate, 15 g of ammonium sulfate, 3 g of sodium lauryl sulfate, 27 g of sodium carboxymethylcellulose, 1 g of disodium edetate, 1 g of a perfume, and 3 g of sodium sulfate.

[Patent Literature 6] EP 1 213 009 A2

Patent literature 6 discloses a "Composition in powder form for bleaching or dyeing of hair and its preparation process" and relates to silicone elastomer-containing powdery cosmetic products and a method for their preparation.

[Patent Literature 7] CN 100 526213 C

Patent literature 7 discloses a "Powdery sodium percarbonate and its preparation method" and relates to a preparation method for the powder sodium percarbonate with active oxygen content at least 10% and 13. 5~15. 2% as the preferred value, which comprises: (A) mixing the anhydrous Na2CO3 and water with some proportion; (B) spraying the H2O2 solution with preferred quantity into the mixture obtained in (A) to make the Na2CO3 and H2O2 reaction fully at 0~70Deg and obtain the slurry of sodium percarbonate; (C) drying with rotation flash evaporation device to obtain the final product. This product is convenient to mix with other constituent, such as detergent and bleacher.

[Patent Literature 8] CN 101 337 658 A

Patent literature 8 discloses a "Method for synthesizing sodium percarbonate of high stability" and relates to a production method for synthesizing sodium percarbonate of high stability is a technology for producing an oxygen containing bleaching agent, and overcomes the shortcoming existing in other methods. The method comprises the following procedures: the first step, refining raw materials; the second step, preparing hydrogen peroxide; the third step, reacting and crystallizing; the forth step, centrifuging; the fifth step, coating with films and baking; the sixth step, packaging finished products; and measuring and separately packaging baked and qualified sodium percarbonate according to requirements of a user. The sodium percarbonate of high stability has main properties as follows: the content of active oxygen is 13.0 to 14.2 percent; the bulk density is 900 to 1100g/L; the moisture content is less than or equal to 2.0 percent; iron is less than or equal to 15ppm; the thermal stability is higher than or equal to 94.0%; and the moisture stability is higher than or equal to 65.0%.

[Patent Literature 9] DE 195 33 524

Patent literature 9 discloses a "Process for the production of sodium percarbonate" and concerns a flexible process for the production of homogenous sodium percarbonate particle or granules. According to the invention, an aqueous solution comprising up to 50 % by weight hydrogen peroxide and solid soda is used to prepare a reaction mash with a medium to high solid content. This reaction mash, which may if necessary be reduced by wet crushing to a fine powder, is then immediately subjected to short-term drying, preferably spray-drying, and possibly also granulation. This process makes it possible efficiently to produce homogeneous sodium percarbonate particles or granules with a variable active oxygen content of between 10 and 14.5 % by weight and of a wide range of particle size and bulk density.

[Patent Literature 10] WO 02 051745

Patent literature 10 discloses a "method for increasing the inner stability of sodium percarbonate" and relates to a method for increasing the inner stability of encapsulated and non-encapsulated sodium percarbonate. According to the invention, particulate, dry sodium percarbonate is treated for at least 2 minutes at a temperature of 70 to 120 DEG C and during the treatment, the air surrounding the particles is exchanged, in order to maintain or reduce humidity.

[Patent Literature 11] EP 1 447 380 A1

Patent literature 11 discloses a "Process for preparing granular sodium percarbonate" and relates to a process for preparing granular sodium percarbonate. More particularly, while the conventional dry process for granular sodium percarbonate performed in such a manner that hydrogen peroxide solution is sprayed to anhydrous sodium carbonate to produce sodium percarbonate and sodium percarbonate produced is then fluidized-dried, produces sodium percarbonate of which particle size is dependent on the particle size of sodium carbonate used and thus a separate granulation step is required, the present process performed in such a manner that hydrogen peroxide solution is sprayed to anhydrous sodium carbonate containing 5-10% water based on the weight of anhydrous sodium carbonate simultaneously with introducing air at a predetermined temperature and rate,; allows for the adjustment of the particle size of sodium percarbonate produced irrespective of the particle size of sodium carbonate used, so that granular sodium percarbonate with desired physical properties can be obtained without performing granulation.

[Patent Literature 12] FR-A-2 924 123

Patent literature 12 discloses a "Detergent product, useful to prepare washing powder, preferably for linen, comprises packaging (film of polymer) and detergent composition having product from Sapindus fruit and stain removing- and bleaching- agent (e.g. sodium carbonate)". The detergent product comprises a packaging and a detergent composition contained in the packaging, where the composition comprises 10-40 wt.% of a product obtained from Sapindusfruit and 60-90 wt. % of a stain removing agent and bleaching agent, the stain removing agent and the bleaching agent is sodium percarbonate, sodium carbonate and/or sodium bicarbonate, the packaging is a polymer film, and the polymer is polypropylene and/or polyethylene. An independent claim is included for a process for producing a cleansing towelette, comprising using the product in at least one washing step, extracting the detergent composition from the packaging and recovering the packaging.

[Patent Literature 13] WPI abstract of JP 11 172286 A

Patent literature 13 discloses a "Bleaching composition-charged container -used for bleaching fabrics" and concerns a bleaching composition-charged container including a plastic container having a ventilation hole, a bleaching agent charged in the container and containing sodium percarbonate and sodium carbonate, wherein the plastic container is made of at least one kind of a linear, low density polyethylene, a low-density polyethylene, a non-extended polypropylene, an extended nylon, an extended polypropylene and a polyethylene terephthalate, and when the container has the wall thickness of 5 to 300 mu , the volume of 0.5 liter, the diameter of the ventilation hole of 0.3 mm, and 400 9 of the bleaching composition is charged into the container, the decomposing speed constant of the sodium percarbonate is 0.4 x 10-8 to 4 x 10-8 sec. -1 in storage at 40 [deg]C and a relative humidity of 80% for 2 weeks.

Generally, powder hair dyes are conveniently transportable and portable and can be used in a very simple manner, advantageously. On comparison with cream or liquid hair dyes, however, powder hair dyes have a drawback of poor hair feeling after application.

Furthermore, solid oxidizing agents are used in powder hair dyes. For example, persulfate salts and percarbonate salts are known as such solid oxidizing agents. Because persulfate salts have strong oxidation potential among others, possibly, persulfate salts may oxidize and decompose oxidized dye polymers indispensable for hair dyeing. Since the oxidation potential of percarbonate salts is poor, alternatively, percarbonate salts less potentially oxidize and decompose such dyes. However, sufficient dyeability or bleaching ability cannot be obtained from percarbonate salts, disadvantageously.

Meanwhile, solid oxidizing agents are more or less decomposed over time, to generate oxygen gas. When powder hair dye compositions are stored in a plastic container, oxygen gas generated via the decomposition of such solid oxidizing agents expands the container, potentially causing the explosion of the container. As a countermeasure against such problem, it is proposed to use a storage container comprising a material with high gas permeability. In that case, however, the oxidation dye subliming inside the storage container turns readily permeable, so that a paper box placing therein the storage container or an attached instruction manual therein may be stained and colored with the subliming oxidation dye. Since water outside the storage container such as atmospheric moisture(water vapor) permeates therein, the contents therein may be moistened even during storage, so that the oxidation dye and the oxidizing agent therein fall in states at poorer stability disadvantageously that the color of the dyed hair may be deteriorated or the dyeing performance may be deteriorated, due to the moistening. The package described in Patent literature 3 is arranged with an oxygen absorption layer (2) outside the oxygen permeation layer (1), to prevent the oxidation and deterioration of the contents due to the oxygen gas generated from such peroxides and also prevent the expansion and explosion of the container. The gas barrier layer(3) provided further outside may have an effect of suppressing the permeation of the subliming oxidation dye to the outside of the storage container or the permeation of moisture from the outside of the storage container into the inside of the storage container. However, apparently, the package described in Patent Literature 3 is significantly highly costly because a unique material of a resin composition in blend with a deoxygen composition of iron series is used in the oxygen absorption layer (2) and the package is of a three-layer structure comprising resin layers with different compositions and features.

Summary of the Invention

It is an object of the invention to enhance the dyeability and bleaching ability of a powder hair dye composition and to get excellent hair feeling after application of the powder hair dye composition. It is an additional object of the invention to suppress the increase of oxygen gas pressure causing the expansion and explosion of a storage package placing therein the powder hair dye composition. It is a still additional object of the invention to prevent a paper box placing therein the storage package or an instruction manual from staining, due to the sublimation of an oxidation dye. Still more additionally, it is an object of the invention to securely retain the excellent storage stability of the powder hair dye composition in the storage package, by using a storage container which allows the oxygen gas inside the container to permeate outside the container and suppresses the permeation of moisture from the outside of the container into the inside of the container.

(First aspect)

In a first aspect of the invention, a storage package obtained by placing a powder hair dye composition for dyeing hair in a storage container has a container wall of a single-layer structure or a multi-layer structure comprising a material selected from polyethylene and polypropylene, wherein the powder hair dye composition for dyeing hair contains sodium percarbonate within a range of the active oxygen amount at 12.0 to 15.3 % by mass as a component (A) and additionally contains the sulfate salt of an oxidation dye as a component (C).

In the first aspect of the invention, the powder hair dye composition contains the component (A), namely powder sodium percarbonate within a range of the active oxygen amount at 12.0 to 15.3 % by mass as an oxidizing agent.

Since common percarbonate salts with the active oxygen amount at less than 12.0 % by mass have poor oxidation potential, sufficient dyeability or bleaching ability cannot be obtained from such percarbonate salts. When the active oxygen amount from sodium percarbonate amounts to 12.0 % by mass or more, however, the oxidation potential is enhanced so that sufficient dyeability and bleaching ability can be exerted.

At the hair application of a powder hair dye composition, powder sodium percarbonate mixed with water is decomposed according to the following chemical formula 1.

$$Na_2CO_3 \cdot 3/2(H_2O_2) \rightarrow Na_2CO_3 + 3/2 \, (H_2O) + 3/4 \, (O_2) \qquad \text{(Chemical formula 1)}$$

Sodium carbonate ($Na_2CO_3$) generated according to the chemical formula 1 interacts as an alkaline agent generally essential for oxidation hair dyes and hair bleaches. Therefore, the amount of a powder alkaline agent to be blended in the powder hair dye composition in accordance with the invention can be reduced. Furthermore, sodium carbonate is such a mild alkaline agent that the resulting hair feeling is not so much poor. Accordingly, the general drawback of powder hair dye compositions, namely poor hair feeling after application thereof can substantially be reduced, so the hair feeling after application of the hair dye is great.

The powder hair dye composition in accordance with the first aspect additionally contains the sulfate salt of an oxidation dye as the component (C).

The sulfate salt of a powder oxidation dye as contained as the component (C) has a hardly subliming property. Even when the powder hair dye composition is placed in a storage container comprising a gas-permeable material described below, the risk of staining (coloring) a paper box placing therein the storage container or an attached instruction manual with the oxidation dye subliming and permeating through the container wall of the storage container is reduced. When the sulfate salt thereof is used as the component (C), further, disadvantages such as oxidation of the oxidation dye under storage due to oxygen and the like or the occurrence of the darkened appearance and the like are prevented. Thus, the storage stability of the oxidation dye gets very great.

Polyethylene and polypropylene are both materials with good gas permeability. The container wall of a single-layer structure or a multi-layer structure according to the first aspect of the invention comprising these materials allows oxygen gas generated via the decomposition of sodium percarbonate to permeate outside the storage container. Therefore, there is no risk of the expansion or explosion of the storage container through the increase of oxygen gas pressure.

(Second aspect)

In a second aspect of the invention, the content of the component (A) in the powder hair dye composition(s) in the storage package of the first aspect is within a range of 20 to 50 % by mass.

In the second aspect, a preferable mode for carrying out the invention is provided, where the content of the component (A) in the powder hair dye composition in the first aspect is within a range of 20 to 50 % by mass. When the content thereof is less than 20 % by mass, the advantage of the first aspect described above may potentially be deteriorated. When the content thereof is more than 50 % by mass, the resulting hair feeling may potentially be poor.

(Third aspect)

In a third aspect of the invention, the powder hair dye composition within the storage package of the first aspect or in the second aspect additionally contains a sequestering agent (metal-sealing agent) as the component (B).

Generally, metal ions deteriorate the dyeability of hair dyes. Additionally, metal ions give roughness with poor feeling to hair. When a sequestering agent in powder is contained in a hair dye using sodium percarbonate as an oxidizing agent, sodium ion derived from sodium percarbonate is captured with the sequestering agent for turning the sodium ion to a harmless material. Consequently, the deterioration of the dyeability and hair feeling during the use of the powder hair dye composition can be prevented more effectively.

(Fourth aspect)

In a fourth aspect of the invention, the content of the component (B) in the powder hair dye composition within the storage package of the third aspect of the invention is within a range of 2.5 to 10 % by mass.

In the fourth aspect, a preferable mode for carrying out the invention is provided, where the content of the component (B) in the powder hair dye composition is within a range of 2.5 to 10 % by mass. When the content thereof is less than 2.5 % by mass, the effect described in the third aspect may potentially be insufficient. When the content is more than 10 % by mass, it cannot be expected that the component (B) would additionally enhance the effect.

(Fifth aspect)

In a fifth aspect of the invention, the component (B) in the powder hair dye composition within the storage package of the third aspect or the fourth aspect is at least one or more selected from disodium edetate, trisodium edetate, ethylenediaminetetraacetic acid, trisodium ethylenediaminehydroxyethyltriacetate, and diethylenetriaminepentaacetic acid.

In the fifth aspect, a particularly preferable mode for carrying out the invention is provided, where the type of the component (B) contained in the powder hair dye composition is specified.

(Sixth aspect)

In a sixth aspect of the invention, the ratio in mass of the content of the component (A) and the content of the component (B), namely (A) / (B) in the powder hair dye composition within the storage package in accordance with any of the third to fifth aspects is within a range of 2.0 to 16.0.

The ratio in mass of the content of sodium percarbonate as the component (A) and the content of a sequestering agent as the component (B), namely (A)/(B) in the powder hair dye composition is within a given preferable range for the exertion of the effect in good balance. In accordance with the sixth aspect, a preferable mode for carrying out the invention is provided, where the ratio in mass namely (A)/(B) is within a range of 2.0 to 16.0. When the ratio in mass namely (A)/(B) is outside the range of 2.0 to 16.0, potentially, the effect of improving the feeling may get insufficient.

(Seventh aspect)

In an seventh aspect of the invention, the component (C) in the powder hair dye composition within the storage package in accordance with the first aspect is at least one or more selected from the following group (c1) or is a combination of at least one or more selected from the following group (c1) and at least one or more selected from the following group (c2):

(c1) p-phenylenediamine sulfate, toluene-2,5-diamine sulfate, p-aminophenol sulfate, o-aminophenol sulfate, 2,2'-[(4-aminophenyl)imino]bisethanol sulfate, 1-hydroxyethyl-4,5-diaminopyrazole sulfate; and
(c2) 5-amino-o-cresol sulfate, m-aminophenol sulfate, 2,6-diaminopyridine sulfate, 2,4-diaminophenoxyethanol sulfate, p-methylaminophenol sulfate.

The type of the component (C) in the powder hair dye composition is preferably the one defined in the seventh aspect. The component (C) as one or more selected from the group (c1) is a primary intermediate, while the component (C) as one or more selected from the group (c2) is a coupler. In other words, satisfactorily, the component (C) may be a primary intermediate alone or may comprise a primary intermediate and a coupler.

(Eighth aspect)

In a eights aspect of the invention, the content of the component (C) in the powder hair dye composition within the storage package in accordance with the sixth or seventh aspect is within a range of 0.25 to 40 % by mass.

In the eights aspect, a preferable mode for carrying out the invention is provided, where the content of the component (C) in the powder hair dye composition is within a range of 0.25 to 40 % by mass. When the content thereof is less than 0.25 % by mass, dyeing cannot be done sufficiently due to the insufficiency of the dye component. When the content is more than 40 % by mass, it cannot be expected that the component (C) would additionally enhance the effect of enhancing the dyeing performance.

(Ninth aspect)

In a ninth aspect of the invention, the ratio in mass of the content of the component (A) and the content of the component (C), namely (A)/(C) in the powder hair dye composition within the storage package in accordance with any of the sixth to eighth aspects is within a range of 0.5 to 160.

The ratio in mass of the content of sodium percarbonate as the component (A) and the content of the sulfate salt of an oxidation dye as the component (C), namely (A) / (C) in the powder hair dye composition is constantly within a given preferable range for the exertion of the effect in good balance. In accordance with the ninth aspect, a preferable mode for carrying out the invention is provided, where the ratio in mass namely (A) / (C) is within a range of 0.5 to 160. When the ratio in mass namely (A) / (C) is outside the range of 0.5 to 160, potentially, the effect of enhancing the dyeing performance may not be expected.

(Tenth aspect)

In an tenth aspect of the invention, the ratio in mass of the content of the component (B) and the content of the component (C), namely (B) / (C) in the powder hair dye composition within the storage package in accordance with any of the sixth to ninth aspects is within a range of 0.07 to 35.

The ratio in mass of the content of a sequestering agent as the component (B) and the content of the sulfate salt of an oxidation dye as the component (C), namely (B) / (C) in the powder hair dye composition is within a given preferable range for the exertion of the effect in good balance. In accordance with the tenth aspect, a preferable mode for carrying out the invention is provided, where the ratio in mass namely (B) / (C) is within a range of 0.07 to 35. When the ratio in mass namely (B)/(C) is outside the range of 0.07 to 35, potentially, the effect of enhancing the dyeing performance may not be expected.

(Eleventh aspect)

In a eleventh aspect of the invention, the thickness of the container wall of the storage container in accordance with the first aspect is within a range of 0.5 to 2.0 mm.

When the thickness of the container wall of the storage container of a single-layer structure or a multi-layer structure is within a range of 0.5 to 2.0 mm, the following effects can be expected. Specifically, oxygen gas in the storage container is allowed to permeate outside the container, while the permeation of water from the outside of the storage container into the inside of the container can be suppressed. Hence, no disadvantage occurs such that the contents are moistened to deteriorate the stability of an oxidation dye or an oxidizing agent therein, although the hair dye composition is not in use. Even when the oxidation dye sublimes to a certain extent inside the storage container, the oxidation dye cannot permeate through the container wall, so that the paper box placing therein the storage container or an attached instruction manual is never stained.

When the thickness of the container wall of the storage container is less than 0.5 mm, the permeability gets too excessively large, so that a concern arises that sufficient suppression of the permeability of moisture from the outside of the storage container into the inside of the container gets hard. When the thickness of the container wall of the storage container is more than 2.0 mm, the permeability gets too excessively small, so that there causes a concern that it is hard to get free the oxygen gas generated inside the storage container outside the container.

(Twelfth aspect)

In a twelfs aspect of the invention, the ratio (X)/(Y) under provisions that the numerical figure (X) is in unit "% by mass" of the content of the component (A) in the powder hair dye composition and the numerical figure (Y) is in unit "mm" of the thickness of the container wall of the storage container in the storage package in the eleventh aspect is within a range of 10 to 80.

The ratio (X)/(Y) under provisions that the numerical figure (X) is in unit "% by mass" of the content of the component (A) in the powder hair dye composition and the numerical figure (Y) is in unit "mm" of the thickness of the container wall of the storage container is preferably within a given range for the exertion of the effect in good balance. In the twelfth aspect, a preferable mode for carrying out the invention is provided, where the ratio (X) / (Y) is within a range of 10 to 80. When the ratio (X)/(Y) is less than 10, potentially, the inner pressure inside the storage package may increase, while the stability of the percarbonate salt may potentially be deteriorated when the ratio (X)/(Y) is more than 80.

Best Mode for Carrying out the Invention

Modes for carrying out the first to twelfth aspects of the invention are described below, including the best embodiments thereof.

[Powder hair dye composition]

The powder hair dye composition is a powder composition for dyeing hair and includes but is not limited to powder hair dye compositions for hair dyeing or bleaching on the basis of the action of the oxidizing agent (sodium percarbonate). More specifically, the powder hair dye composition includes at least a powder oxidation hair dye composition to be used for the purpose of hair dyeing and a powder hair bleach composition to be used for the purpose of hair bleaching.

The powder oxidation hair dye composition contains at least the oxidizing agent (sodium percarbonate) and an oxidation dye and generally contains an alkaline agent. The oxidation hair dye composition may be in blend with a direct dye. The oxidation dye comprises a primary intermediate and a coupler or comprises a primary intermediate. The powder hair bleach composition contains at least the oxidizing agent (sodium percarbonate) and generally contains an alkaline agent.

In using the powder hair dye composition in a storage container of the invention, the powder hair dye composition is dissolved in an appropriate solution for the dissolution such as water to prepare a chemical solution in paste, like general powder hair dyes, which is coated on hair, using for example a comb, a brush, or brushes, for hair dyeing or bleaching. The mix ratio of the powder hair dye and the solution for the dissolution is generally about 1:1 to 1:20, preferably 1:2 to 1:10 in mass ratio.

[Major components of the powder hair dye composition]

(Component (A))

The powder hair dye composition of the invention contains solid (powder) sodium percarbonate, namely component (A) as the oxidizing agent, where the active oxygen amount is within a range of 12.0 to 15.3 % by mass.

In accordance with the invention, the active oxygen amount from sodium percarbonate is assayed by the following steps (1) to (4). (1) About 1 g of a sample of sodium percarbonate is weighed; the exact weighed amount thereof is recorded; and the sample is placed in a 200-ml Erlenmeyer flask for dissolution in 100 ml of distilled water. (2) 10 ml of dilute sulfuric acid and 2 g of potassium iodide are added to the resulting solution; then, the Erlenmeyer flask is sealed for thoroughly mixing together the solution; and the flask is left to stand in darkness for 10 minutes. (3) Potassium sulfate is then generated via a neutralization reaction (ion exchange) to get free iodide ion. The iodide ion is oxidized with sodium percarbonate, to generate iodine ($I_2$). (4) The free iodine is titrated with an aqueous 0.1N sodium thiosulfate solution. At the steps (1) to (4), when the amount of weighed sodium percarbonate sample is designated as "a" (mg); the activity coefficient of 0.1N sodium thiosulfate is designated as "f"; and the volume needed for titration is designated as "c" (ml), the active oxygen amount (%) from the sodium percarbonate sample is represented by the following equation 1.

$$\text{Active oxygen amount (\%)} = (c \times f \times 0.1 \times 8 \times 100)/a$$

$$\dots \text{(Equation 1)}$$

The "activity coefficient f" described above is described below. When the concentrations of solutes in a solution are low, generally, the resulting solution may satisfactorily be designated as an ideal solution. When the concentrations thereof are higher, the interaction of the solutes is no more negligible, so that the physicochemical properties of the resulting solution are deviated from those of the ideal solution. The activity coefficient is a coefficient for correcting the deviation.

The content of the component (A) is not specifically limited but is within a range of 20 to 50 % by mass, preferably 20 to 40 % by mass, in particular.

Generally, the oxidizing agent to be contained in the powder hair dye composition includes for example various perborate salts, various peroxide salts, various sulfate salts added with hydrogen peroxide, various phosphate salts added with hydrogen peroxide, various pyrophosphate salts added with hydrogen peroxide, percarbamide and melamine peroxide, other than sodium percarbonate. Preferably, the powder hair dye composition of the invention never contains any oxidizing agent except sodium percarbonate. However, the powder hair dye composition may possibly contain an oxidizing agent other than sodium percarbonate, along with sodium percarbonate.

(Component (B))

The powder hair dye composition of the invention preferably contains additionally a sequestering agent as the component (B).

The content of the component (B) is not specifically limited but is preferably within a range of 2. 5 to 10 % by mass, particularly 3.0 to 8. 0 % by mass. The mass ratio of the content of the component (A) and the content of the component (B), namely the mass ratio (A) / (B) of the two is preferably within a range of 2.0 to 16.0, particularly 2.5 to 13 % by mass.

The type of the sequestering agent as the component (B) is not specifically limited. As such, for example, at least one or more selected from disodium edetate, trisodium edetate, ethylenediaminetetraacetic acid, trisodium ethylenediaminehydroxyethyltriacetate, and diethylenetriaminepentaacetic acid can preferably be used. Additionally, various known sequestering agents for example sodium citrate, sodium metaphosphate, and sodium polyphosphate may also be used.

(Component (C))

When the powder hair dye composition in the storage container of the invention is a powder oxidation hair dye composition, additionally, the powder hair dye composition preferably contains the sulfate salt of an oxidation dye as the component (C). Preferably, the powder hair dye composition never contains any oxidation dye in compound forms except the form of the sulfate salt.

The content of the component (C) is not specifically limited. The content is preferably within a range of 0.25 to 40 % by mass, particularly 0.5 to 35 % by mass. Further, the mass ratio of the content of the component (A) and the content of the component (C), namely the mass ratio (A) / (C) of the two, is preferably within a range of 0.5 to 160, particularly 0.6 to 80. Furthermore, the mass ratio of the content of the component (B) and the content of the component (C), namely the mass ratio (B)/(C) of the two, is preferably within a range of 0.07 to 35, particularly 0.08 to 16.

As the sulfate salt of an oxidation dye, the powder hair dye composition may contain only the sulfate salt of a primary intermediate but may satisfactorily contain the sulfate salt of a primary intermediate and the sulfate salt of a coupler.

Regarding the type of the sulfate salt of an oxidation dye, the type of the sulfate salt of a primary intermediate and the type of the sulfate salt of a coupler are not specifically limited. As such, at least one or more selected from the following group (c1) or a combination of at least one or more selected from the following group (c1) and at least one or more selected from the following group (c2) is particularly preferable. The group (c1) includes the sulfate salts of primary intermediates, while the group (c2) includes the sulfate salts of couplers:

(c1) p-phenylenediamine sulfate, toluene-2,5-diamine sulfate, p-aminophenol sulfate, o-aminophenol sulfate, 2,2'-[(4-aminophenyl)imino]bisethanol sulfate, 1-hydroxyethyl-4,5-diaminopyrazole sulfate; and
(c2) 5-amino-o-cresol sulfate, m-aminophenol sulfate, 2,6-diaminopyridine sulfate, 2,4-diaminophenoxyethanol sulfate, p-methylaminophenol sulfate.

As the dye component in the powder hair dye composition of the invention, still further, known direct dyes such as nitro-series dyes, azo dyes, nitroso dyes, triphenylmethane dyes, xanthene dyes, quinoline dyes, anthraquinone dyes and indigo dyes may be blended therein other than the component (C).

[Other components in the powder hair dye composition]

For the purpose of enhancing the dyeability, generally, an alkaline agent is blended in the powder hair dye composition of the invention. The alkaline agent includes for example sodium carbonate, magnesium carbonate, sodium metasilicate, and basic amino acids.

Additionally, components other than those described above including for example dispersants, oily matters, sodium sulfate, thickeners, surfactants, polypeptides, and pH adjusters may be selected and then blended at an appropriate amount in the powder hair dye composition of the invention, as long as the selected components never disadvantageously affect the advantages of the invention.

The dispersants include for example metal salts of stearic acid, silicic acid, metal salts of silicic acid, talc, sucrose fatty acid esters, crystalline cellulose, hydroxypropylcellulose at a low substitution degree, dextrin, starch and lactose. As the dispersants, metal salts of stearic acid are particularly preferable. The preferable content of such dispersants is about 0.05 to 20 % by mass in the composition.

As the oily matters, liquids with fluidity at normal temperature are preferably used. The oily matters include for example α-olefin oligomer, light isoparaffin, light liquid isoparaffin, squalene, synthetic squalene, squalene derived from plants, liquid isoparaffin, liquid petrolatum, and silicone oil. The preferable content of such oily matters is about 0.01 to 2.0 % by mass in the composition.

For preventing the deterioration of the dyeability of the powder hair dye composition due to moisture absorption, sodium sulfate is blended. Sodium sulfate is blended at an amount appropriately determined.

The thickeners include for example seaweeds, sodium alginate, methylcellulose, hydroxyethylcellulose, sodium carboxymethylcellulose, polyvinyl alcohol, polyvinylpyrrolidone, sodium polyacrylate, guar gum, xanthane gum, quaternary nitrogen-containing cellulose ether.

The surfactants include for example higher alkylbenzene sulfonate salts, fatty acid soaps, higher alkylsulfonate salts, and higher alkylphosphate esters.

The polypeptides are blended therein for hair protection. The polypeptides include for example hydrolyzed products prepared by treating proteins such as collagen, keratin, elastin, fibroin, conchiolin, soybean protein, and casein with for example acids, alkalis and enzymes, and additionally include for example cation-modified proteins prepared as quaternary compounds.

The pH adjusters include for example ammonium sulfate, ammonium nitrate, ammonium carbonate, ammonium chloride, ammonium dihydrogen phosphate, diammonium hydrogen phosphate, citric acid, tartaric acid, lactic acid and succinic acid.

As solid optional components, further, antistatic agents, hair softeners, antioxidants, preservatives, excipients, dyes and flavors and the like may also be blended in the powder hair dye composition of the invention.

The powder hair dye composition is produced by drying and mixing together the individual raw material powders excluding the oily matters described above and then spraying the oily matters to the resulting dry mixture.

[Storage package]

The storage package of the invention is prepared by placing the powder hair dye composition in a storage container of a single-layer structure or a multi-layer structure comprising a material selected from polyethylene and polypropylene.

The storage container comprises at least a container body and an opening/closing unit at the opening of the container body, and when the opening/closing unit is closed, the contents in the container body are sealed in an air-tight manner. Depending on the need, the shape and volume of the container should be freely designed, with no specific limitation.

The storage container has a container wall of a single-layer structure comprising polyethylene (PE) or polypropylene (PP) layer with good gas permeability or has a multi-layer structure comprising two layers or more selected from polyethylene layers and polypropylene layers. From the standpoint of production cost, the storage container preferably has a container wall of a single-layer structure comprising polyethylene layer or polypropylene layer.

The thickness of the container wall of the storage container, which is in a single-layer structure or a multi-layer structure, is not basically limited, but is preferably within a range of 0.5 to 2.0 mm due to the reason described in relation with the eleventh aspect. Further, the numerical figure (Y) in unit "mm" of the thickness of the container wall of the storage container and the numerical figure (X) in unit "% by mass" of the content of the component (A) in the powder hair dye composition as the contents in the storage container satisfy the ratio of the two, namely (X)/(Y) such that the ratio is preferably within a range of 10 to 80, particularly 13 to 67, due to the reason described in relation with the twelfth aspect.

Examples

Examples of the invention and Comparative Examples are described below. The technical scope of the invention is never limited by the following Examples and Comparative Examples.

Powder hair dye compositions of Examples 1 to 41, as shown in Tables 1 to 5 in a latter part and those of Comparative Examples 1 to 16 as shown in Tables 6 and 7 in the latter part were prepared. Components expressed by Component 1 to Component 33 commonly described in the column "Component" in Tables 1 to 7 and Components expressed by Component 34 and Component 35 described in the column "Component" in Tables 6 and 7 individually represent the following Components. All the Components are powdery.

Component 1: sodium percarbonate
Component 2: sodium carbonate
Component 3: sodium phosphate
Component 4: ammonium sulfate
Component 5: sodium carboxymethylcellulose
Component 6: disodium lauryl sulfosuccinate
Component 7: o-[2-hydroxy-3-(trimethylammonio)propyl]guar gum chloride
Component 8: magnesium stearate
Component 9: titanium dioxide
Component 10: tartaric acid
Component 11: trisodium ethylenediaminehydroxyethyltriacetate
Component 12: disodium edetate
Component 13: trisodium edetate
Component 14: ethylenediaminetetraacetic acid
Component 15: diethylenetriaminepentaacetic acid
Component 16: flavor
Component 17: $\beta$-cyclodextrin
Component 18: sodium sulfate
Component 19: potato starch
Component 20: p-phenylenediamine sulfate
Component 21: p-phenylenediamine
Component 22: toluene-2,5-diamine sulfate
Component 23: p-aminophenol sulfate
Component 24: p-aminophenol
Component 25: o-aminophenol sulfate
Component 26: 2,2'-[(4-aminophenyl)imino]bisethanol sulfate
Component 27: 1-hydroxyethyl-4,5-diaminopyrazole sulfate
Component 28: 5-amino-o-cresol sulfate
Component 29: m-aminophenol sulfate
Component 30: 2,6-diaminopyridine sulfate
Component 31: 2,4-diaminophenoxyethanol sulfate
Component 32: p-methylaminophenol sulfate.
Component 33: resorcin • $\beta$-cyclodextrin mixture
Component 34: 5-amino-o-cresol
Component 35: m-aminophenol

Examples 1-15, 32-33 and 25-27 are not part of the invention as claimed (no sulfate salt of oxydation dye and/or no wall made of PE or PP).

Component attached with the expression "(A)" after Component Nos. are sodium percarbonate with the active oxygen amount within a range of 12.0 to 15.3 % by mass; Components attached with the expression "(A comparative)" mean sodium percarbonate with the active oxygen amount outside the range of 12.0 to 15.3 % by mass. Components attached with the expression "(B)" mean the component (B); Components attached with the expression "(c1)" or "(c2)" mean a component of (c1) or a component of (c2).

The numerical figure representing the content of each of the components in powder in the tables is expressed in "% by mass". The numerical figure in parenthesis immediately after the numerical figures expressing the component of the component (A) or the component (A comparative) expresses the active oxygen amount from the component (A) or the component (A comparative).

Among the descriptions shown below the frames of the individual tables, " (C) " means "(c1) + (c2)". Below the frames of the individual tables, further, parameters "(A) / (B)", "(A) / (C)" and "(B) / (C)" are shown. The parameter "(X) / (Y)" as the ratio between the numerical figure (X) in unit "% by mass" of the content of the component (A) and the numerical figure (Y) in unit "mm" of the thickness of the container wall of the storage container described below is additionally shown.

(Evaluation of dyeability)

After 50 ml of water was added to and mixed in 10 g of each sample of the powder hair dye compositions in the individual Examples and the individual Comparative Examples immediately after their preparation, the resulting mixture was applied to gray hair tufts for tests for hair dyeing. After the completion of hair dyeing, 10 panelists evaluated the hair tufts under visual observation, for ranking on the straight choice standard of "good dyeability" or "not good dyeability". The results of the evaluation are expressed as follows: a powder hair dye composition evaluated by 8 or more panelists out of the 10 panelists as "good dyeability" is marked with "5"; a powder hair dye composition evaluated by 6 or 7 panelists as "good dyeability" is marked with "4"; a powder hair dye composition evaluated by 4 or 5 panelists as "good dyeability" is marked with "3"; a powder hair dye composition evaluated by 2 or 3 panelists as "good dyeability" is marked with "2"; a powder hair dye composition evaluated by one panelist or none of the panelist as "good dyeability" is marked with "I". The scores are shown in the column "dyeability" below the frames of the individual tables.

(Evaluation of feeling)

10 panelists evaluated the hair tufts after the completion of dyeing as described in the section "Evaluation of dyeability" described above, for ranking of hair feeling on the straight choice standard of "good feeling" or "not good feeling". The results of the evaluation are expressed as follows: a powder hair dye composition evaluated by 8 or more panelists out of the 10 panelists as "good feeling" is marked with "5"; a powder hair dye composition evaluated by 6 or 7 panelists as "good feeling" is marked with "4"; a powder hair dye composition evaluated by 4 or 5 panelists as "good feeling" is marked with "3"; a powder hair dye composition evaluated by 2 or 3 panelists as "good feeling" is marked with "2"; a powder hair dye composition evaluated by one panelist or none of the panelist as "good feeling" is marked with "1". The scores are shown in the column "hair feeling" below the frames of the individual tables.

(Evaluation of storage stability)

After the preparation of the powder hair dye compositions of the individual Examples and the individual Comparative Examples, the powder hair dye compositions were stored under a condition of 50°C for one month. Using samples after the storage, the storage stability of the samples was evaluated.

After 50 ml of water was added to and mixed in 10 g of each of the samples, the resulting mixture was applied to gray hair tufts for tests for hair dyeing. After the completion of hair dyeing, 10 panelists evaluated the hair tufts treated with the samples after storage under visual observation, on comparison with the hair tufts after completion of hair dyeing with the corresponding hair dye compositions of the Examples and the Comparative Examples (the hair tufts treated with the samples immediately after preparation) as described in the section "Evaluation of dyeability", for ranking on the straight choice standard of "the same level of dyeability" or "not the same level of dyeability". The results of the evaluation are expressed as follows: a powder hair dye composition evaluated by 7 or more panelists out of the 10 panelists as "the same level of dyeability" is marked with "⊙"; a powder hair dye composition evaluated by 5 or 6 panelists as "the same level of dyeability" is marked with "○"; a powder hair dye composition evaluated by 3 or 4 panelists as "the same level of dyeability" is marked with "Δ"; a powder hair dye composition evaluated by 2 or less panelists as "the same level of dyeability" is marked with "X". The markers are shown in the column "storage stability" below the frames of the individual tables.

(Evaluation of inner pressure increase)

After 20 g of each of the powder hair dye compositions of the individual Examples and the individual Comparative Examples was placed in a 30-ml plastic storage container, the container was sealed with a lid for storage under a condition of 50°C for one month. The materials and the layer structures constituting the container walls of the storage containers used in the individual Examples and the individual Comparative Examples are shown in the column "container material" below the frames of the individual tables; the thickness (mm) of each container wall is shown in the column "T". For example, "PE" in the column "container material" means that the container wall is of a polyethylene single-layer structure; "PE/PP" means that the container wall is of a double-layer structure where the inner layer of polyethylene and the outer layer of polypropylene are of the same thickness; and "PVC" means that the container wall is of a single-layer structure of polyvinyl chloride.

The expansion of the storage containers after the termination of storage was evaluated under visual observation. The evaluation was done on the straight choice standard as follows; no expansion of storage container was evaluated as "good"; and any expansion thereof was evaluated as "not good". 10 panelists evaluated the expansion, where the expansion evaluated by 7 or more panelists out of the 10 panelists as "good" is marked with "⊙"; the expansion evaluated by 5 or 6 panelists as "good" is marked with "○"; the expansion evaluated by 3 or 4 panelists as "good" is marked with "△"; the expansion evaluated by 2 or less panelists as "good" is marked with "×". The markers are shown in the column "inner pressure increase" below the frames of the individual tables.

(Results of Evaluation)

Based on the evaluation results of the powder hair dye compositions of the Examples 1 to 41 and the Comparative Examples 1 to 16 as shown in Tables 1 to 7, the following points can mainly be remarked.

1) On comparison of the powder hair dye compositions containing the component (A) of each of the Examples with the powder hair dye compositions containing the component (A comparative) of each of the Comparative Examples, the evaluation results of the dyeability of the powder hair dye compositions of the Examples are entirely great.
2) However, the powder hair dye compositions never containing the component (B) have relatively low dyeability. Particularly, the powder hair dye compositions never containing the component (B) were evaluated as poor hair feeling.
3) The powder hair dye compositions never containing the component (C) in the sulfate salt form as an oxidation dye have poor storage stability.
4) The results of the individual evaluations are significantly different, depending on the satisfaction or no satisfaction of the individual parameters, namely "(A) / (B)" defined in the sixth aspect, "(A)/(C)" defined in the ninth aspect, "(B)/(C)" defined in the tehth aspect, and "(X)/(Y)" defined in the twelfth aspect.
5) The evaluation results of the inner pressure increase are significantly different, depending on the material composing a storage container, which is PVC or which is PE and/or PP.

[Table 1]

| Component | Example 1 | Example 2 | Example 3 | Example 4 | Example 5 | Example 6 | Example 7 |
|---|---|---|---|---|---|---|---|
| Component 1 (A) | 16 (12) | 53 (12) | 25 (12) | 35 (12) | 30 (13) | 30 (13) | 30 (13) |
| Component 2 | | | | | | | |
| Component 3 | | | | | | | |
| Component 4 | 15.00 | 15.00 | 15.00 | 15.00 | 15.00 | 15.00 | 15.00 |
| Component 5 | 17.00 | 17.00 | 17.00 | 17.00 | 17.00 | 17.00 | 17.00 |
| Component 6 | 1.00 | 1.00 | 1.00 | 1.00 | 1.00 | 1.00 | 1.00 |
| Component 7 | 0.50 | 0.50 | 0.50 | 0.50 | 0.50 | 0.50 | 0.50 |
| Component 8 | 0.50 | 0.50 | 0.50 | 0.50 | 0.50 | 0.50 | 0.50 |
| Component 9 | 0.50 | 0.50 | 0.50 | 0.50 | 0.50 | 0.50 | 0.50 |
| Component 10 (B) | | | | | 1.50 | 4.00 | 8.00 |
| Component 11 (B) | | | | | | | |
| Component 12 (B) | | | | | | | |
| Component 13 (B) | | | | | | | |
| Component 14 (B) | | | | | | | |
| Component 15 (B) | | | | | | | |
| Component 16 | 0.50 | 0.50 | 0.50 | 0.50 | 0.50 | 0.50 | 0.50 |
| Component 17 | 0.50 | 0.50 | 0.50 | 0.50 | 0.50 | 0.50 | 0.50 |
| Component 18 | 38.40 | 1.40 | 29.40 | 19.40 | 22.90 | 20.40 | 16.40 |
| Component 19 | 3.00 | 3.00 | 3.00 | 3.00 | 3.00 | 3.00 | 3.00 |

(continued)

| Component | Example 1 | Example 2 | Example 3 | Example 4 | Example 5 | Example 6 | Example 7 |
|---|---|---|---|---|---|---|---|
| Component 20 (c1) | | | | | | | |
| Component 21 | 4.60 | 4.60 | 4.60 | 4.60 | 4.60 | 4.60 | 4.60 |
| Component 22 (c1) | | | | | | | |
| Component 23 (c1) | | | | | | | |
| Component 24 | 2.50 | 2.50 | 2.50 | 2.50 | 2.50 | 2.50 | 2.50 |
| Component 25 (c1) | | | | | | | |
| Component 26 (c1) | | | | | | | |
| Component 27 (c1) | | | | | | | |
| Component 28 (c2) | | | | | | | |
| Component 29 (c2) | | | | | | | |
| Component 30 (c2) | | | | | | | |
| Component 31 (c2) | | | | | | | |
| Component 32 (c2) | | | | | | | |
| Component 33 | | | | | | | |
| Total | 100.00 | 100.00 | 100.00 | 100.00 | 100.00 | 100.00 | 100.00 |
| Total content of (C) | 0.00 | 0.00 | 0.00 | 0.00 | 0.00 | 0.00 | 0.00 |
| Total content of (B) | 0.00 | 0.00 | 0.00 | 0.00 | 1.50 | 4.00 | 8.00 |
| Material of storage container | PE | PE | PP | PE/PP | PE | PE | PE |
| (Y) | 0.30 | 0.30 | 0.30 | 0.30 | 0.30 | 0.30 | 0.30 |
| (A)/(B) | - | - | - | - | 20.00 | 7.50 | 3.75 |
| (A)/(C) | - | - | - | - | - | - | - |
| (B)/(C) | - | - | - | - | - | - | - |
| (X)/(Y) | 53.3 | 176.7 | 83.3 | 116.7 | 100.0 | 100.0 | 100.0 |
| Dyeability | 3 | 3 | 4 | 4 | 4 | 4 | 4 |
| Feeling | 2 | 2 | 2 | 2 | 3 | 4 | 4 |
| Storage stability | × | × | × | × | × | × | × |
| Inner pressure increase | ○ | ○ | ○ | ○ | ○ | ○ | ○ |

[Table 2]

| | Example 8 | Example 9 | Example 10 | Example 11 | Example 12 | Example 13 | Example 14 | Example 15 | Example 16 | Example 17 |
|---|---|---|---|---|---|---|---|---|---|---|
| Component 1 (A) | 30 (13) | 30 (13) | 30 (13) | 30 (13) | 30 (13) | 30 (13) | 30 (13) | 30 (13) | 30 (13) | 22 (13) |
| Component 2 | | | | | | | | | | |
| Component 3 | | | | | | | | | | |
| Component 4 | 15.00 | 15.00 | 15.00 | 15.00 | 15.00 | 15.00 | 15.00 | 15.00 | 15.00 | 15.00 |
| Component 5 | 17.00 | 17.00 | 17.00 | 17.00 | 17.00 | 17.00 | 17.00 | 17.00 | 17.00 | 5.00 |
| Component 6 | 1.00 | 1.00 | 1.00 | 1.00 | 1.00 | 1.00 | 1.00 | 1.00 | 1.00 | 1.00 |
| Component 7 | 0.50 | 0.50 | 0.50 | 0.50 | 0.50 | 0.50 | 0.50 | 0.50 | 0.50 | 0.50 |
| Component 8 | 0.50 | 0.50 | 0.50 | 0.50 | 0.50 | 0.50 | 0.50 | 0.50 | 0.50 | 0.50 |
| Component 9 | 0.50 | 0.50 | 0.50 | 0.50 | 0.50 | 0.50 | 0.50 | 0.50 | 0.50 | 0.50 |
| Component 10 (B) | | | | | | 2.00 | | | | |
| Component 11 (B) | 6.00 | | | | | | | | 5.00 | 5.00 |
| Component 12 (B) | | 6.00 | | | | | | 2.00 | 2.00 | |
| Component 13 (B) | | | 6.00 | | | | | | | |
| Component 14 (B) | | | | 6.00 | | 6.00 | 6.00 | | | |
| Component 15 (B) | | | | | 6.00 | | | 6.00 | | |
| Component 16 | 0.50 | 0.50 | 0.50 | 0.50 | 0.50 | 0.50 | 0.50 | 0.50 | 0.50 | 0.50 |
| Component 17 | 0.50 | 0.50 | 0.50 | 0.50 | 0.50 | 0.50 | 0.50 | 0.50 | 0.50 | 0.50 |
| Component 18 | 18.40 | 18.40 | 18.40 | 18.40 | 18.40 | 16.40 | 16.40 | 16.40 | 26.20 | 1.60 |
| Component 19 | 3.00 | 3.00 | 3.00 | 3.00 | 3.00 | 3.00 | 3.00 | 3.00 | 3.00 | 3.00 |
| Component 20 (c1) | | | | | | | | | 0.30 | 26.00 |
| Component 21 | 4.60 | 4.60 | 4.60 | 4.60 | 4.60 | 4.60 | 4.60 | 4.60 | | |
| Component 22 (c1) | | | | | | | | | | 15.00 |
| Component 23 (c1) | | | | | | | | | | |
| Component 24 | 2.50 | 2.50 | 2.50 | 2.50 | 2.50 | 2.50 | 2.50 | 2.50 | | |
| Component 25 (c1) | | | | | | | | | | |

EP 2 361 604 B1

(continued)

| | Example 8 | Example 9 | Example 10 | Example 11 | Example 12 | Example 13 | Example 14 | Example 15 | Example 16 | Example 17 |
|---|---|---|---|---|---|---|---|---|---|---|
| Component 26 (c1) | | | | | | | | | | |
| Component 27 (c1) | | | | | | | | | | |
| Component 28 (c2) | | | | | | | | | | |
| Component 29 (c2) | | | | | | | | | | 3.90 |
| Component 30 (c2) | | | | | | | | | | |
| Component 31 (c2) | | | | | | | | | | |
| Component 32 (c2) | | | | | | | | | | |
| Component 33 | | | | | | | | | | |
| Total | 100.00 | 100.00 | 100.00 | 100.00 | 100.00 | 100.00 | 100.00 | 100.00 | 100.00 | 100.00 |
| Total content of (C) | 0.00 | 0.00 | 0.00 | 0.00 | 0.00 | 0.00 | 0.00 | 0.00 | 0.30 | 44.90 |
| Total content of (B) | 6.00 | 6.00 | 6.00 | 6.00 | 6.00 | 8.00 | 8.00 | 8.00 | 5.00 | 5.00 |
| Material of storage container | PE | PE | PE | PE | PE | PE | PE | PE | PE | PE |
| (Y) | 0.30 | 0.30 | 0.30 | 0.30 | 0.30 | 0.30 | 0.30 | 0.30 | 0.30 | 0.30 |
| (A)/(B) | 5.00 | 5.00 | 5.00 | 5.00 | 5.00 | 3.75 | 3.75 | 3.75 | 6.00 | 6.00 |
| (A)/(C) | - | - | - | - | - | - | - | - | 100.00 | 0.49 |
| (B)/(C) | - | - | - | - | - | - | - | - | 16.67 | 0.11 |
| (X)/(Y) | 100.0 | 100.0 | 100.0 | 100.0 | 100.0 | 100.0 | 100.0 | 100.0 | 100.0 | 73.3 |
| Dyeability | 5 | 5 | 5 | 5 | 5 | 5 | 5 | 5 | 5 | 5 |
| Feeling | 5 | 5 | 5 | 5 | 5 | 5 | 5 | 5 | 5 | 5 |
| Storage stability | × | × | × | × | × | × | × | × | Δ | Δ |
| Inner pressure increase | ○ | ○ | ○ | ○ | ○ | ○ | ○ | ○ | ○ | ○ |

[Table 3]

| | Example 18 | Example 19 | Example 20 | Example 21 | Example 22 | Example 23 | Example 24 | Example 25 | Example 26 | Example 27 |
|---|---|---|---|---|---|---|---|---|---|---|
| Component 1 (A) | 30 (13) | 30 (13) | 30 (13) | 30 (13) | 30 (13) | 30 (13) | 30 (13) | 16 (13) | 16 (13) | 16 (13) |
| Component 2 | | | | | | | | | | |
| Component 3 | | | | | | | | | | |
| Component 4 | 15.00 | 15.00 | 15.00 | 15.00 | 15.00 | 15.00 | 15.00 | 15.00 | 15.00 | 15.00 |
| Component 5 | 17.00 | 17.00 | 17.00 | 17.00 | 17.00 | 17.00 | 17.00 | 17.00 | 17.00 | 17.00 |
| Component 6 | 1.00 | 1.00 | 1.00 | 1.00 | 1.00 | 1.00 | 1.00 | 1.00 | 1.00 | 1.00 |
| Component 7 | 0.50 | 0.50 | 0.50 | 0.50 | 0.50 | 0.50 | 0.50 | 0.50 | 0.50 | 0.50 |
| Component 8 | 0.50 | 0.50 | 0.50 | 0.50 | 0.50 | 0.50 | 0.50 | 0.50 | 0.50 | 0.50 |
| Component 9 | 0.50 | 0.50 | 0.50 | 0.50 | 0.50 | 0.50 | 0.50 | 0.50 | 0.50 | 0.50 |
| Component 10 (B) | | | | | | | | 1.50 | 5.00 | 5.00 |
| Component 11 (B) | 5.00 | 5.00 | 5.00 | 5.00 | 5.00 | 5.00 | 5.00 | | | |
| Component 12 (B) | | | | | | | | | | |
| Component 13 (B) | | | | | | | | | | |
| Component 14 (B) | | | | | | | | | | |
| Component 15 (B) | | | | | | | | | | |
| Component 16 | 0.50 | 0.50 | 0.50 | 0.50 | 0.50 | 0.50 | 0.50 | 0.50 | 0.50 | 0.50 |
| Component 17 | 0.50 | 0.50 | 0.50 | 0.50 | 0.50 | 0.50 | 0.50 | 0.50 | 0.50 | 0.50 |
| Component 18 | 17.90 | 17.90 | 17.90 | 17.90 | 17.90 | 17.90 | 17.90 | 36.90 | 33.40 | 40.50 |
| Component 19 | 3.00 | 3.00 | 3.00 | 3.00 | 3.00 | 3.00 | 3.00 | 3.00 | 3.00 | 3.00 |
| Component 20 (c1) | 4.60 | 4.60 | 4.60 | 4.60 | 4.60 | 4.60 | 4.60 | | | |
| Component 21 | | | | | | | | 4.60 | 4.60 | |
| Component 22 (c1) | | | | | | | | | | |
| Component 23 (c1) | 2.50 | 2.50 | 2.50 | 2.50 | 2.50 | 2.50 | 2.50 | | | |
| Component 24 | | | | | | | | 2.50 | 2.50 | |
| Component 25 (c1) | | | | | | | | | | |

EP 2 361 604 B1

(continued)

| | Example 18 | Example 19 | Example 20 | Example 21 | Example 22 | Example 23 | Example 24 | Example 25 | Example 26 | Example 27 |
|---|---|---|---|---|---|---|---|---|---|---|
| Component 26 (c1) | | | | | | | | | | |
| Component 27 (c1) | | | | | | | | | | |
| Component 28 (c2) | 0.80 | 0.80 | 0.80 | 0.80 | 0.80 | 0.80 | 0.80 | | | |
| component 29 (c2) | 0.70 | 0.70 | 0.70 | 0.70 | 0.70 | 0.70 | 0.70 | | | |
| Component 30 (c2) | | | | | | | | | | |
| Component 31 (c2) | | | | | | | | | | |
| Component 32 (c2) | | | | | | | | | | |
| Component 33 | | | | | | | | | | |
| Total | 100.00 | 100.00 | 100.00 | 100.00 | 100.00 | 100.00 | 100.00 | 100.00 | 100.00 | 100.00 |
| Total content of (C) | 8.60 | 8.60 | 8.60 | 8.60 | 8.60 | 8.60 | 8.60 | 0.00 | 0.00 | 0.00 |
| Total content of (B) | 5.00 | 5.00 | 5.00 | 5.00 | 5.00 | 5.00 | 5.00 | 1.50 | 5.00 | 5.00 |
| Material of storage container | PE | PE | PP | PE | PE | PP | PE/PP | PE | PE | PE |
| (Y) | 0.30 | 2.50 | 0.80 | 0.80 | 0.80 | 0.80 | 0.80 | 0.30 | 0.30 | 0.30 |
| (A)/(B) | 6.00 | 6.00 | 6.00 | 6.00 | 6.00 | 6.00 | 6.00 | 10.67 | 3.20 | 3.20 |
| (A)/(C) | 3.49 | 3.49 | 3.49 | 3.49 | 3.49 | 3.49 | 3.49 | - | - | - |
| (B)/(C) | 0.58 | 0.58 | 0.58 | 0.58 | 0.58 | 0.58 | 0.58 | - | - | - |
| (X)/(Y) | 100.0 | 12.0 | 37.5 | 37.5 | 37.5 | 37.5 | 37.5 | 53.3 | 53.3 | 53.3 |
| Dyeability | 5 | 5 | 5 | 5 | 5 | 5 | 5 | 4 | 4 | 1 |
| Feeling | 5 | 5 | 5 | 5 | 5 | 5 | 5 | 3 | 4 | 3 |
| Storage stability | ○ | ○ | ○ | ⊙ | ⊙ | ⊙ | ⊙ | × | × | - |
| Inner pressure increase | ○ | ○ | ⊙ | ⊙ | ⊙ | ⊙ | ⊙ | ○ | ○ | ○ |

[Table 4]

| | Example 28 | Example 29 | Example 30 | Example 31 | Example 32 | Example 33 |
|---|---|---|---|---|---|---|
| Component 1 (A) | 16 (13) | 16 (13) | 16 (13) | 16 (13) | 16 (13) | 16 (13) |
| Component 2 | | | | | | |
| Component 3 | | | | | | |
| Component 4 | 15.00 | 15.00 | 15.00 | 15.00 | 15.00 | 15.00 |
| Component 5 | 17.00 | 17.00 | 17.00 | 17.00 | 17.00 | 17.00 |
| Component 6 | 1.00 | 1.00 | 1.00 | 1.00 | 1.00 | 1.00 |
| Component 7 | 0.50 | 0.50 | 0.50 | 0.50 | 0.50 | 0.50 |
| Component 8 | 0.50 | 0.50 | 0.50 | 0.50 | 0.50 | 0.50 |
| Component 9 | 0.50 | 0.50 | 0.50 | 0.50 | 0.50 | 0.50 |
| Component 10 (B) | | | | | | |
| Component 11 (B) | | | | | | |
| Component 12 (B) | | | | | | |
| Component 13 (B) | | | | | | |
| Component 14 (B) | | | | | | |
| Component 15 (B) | | | | | | |
| Component 16 | 0.50 | 0.50 | 0.50 | 0.50 | 0.50 | 0.50 |
| Component 17 | 0.50 | 0.50 | 0.50 | 0.50 | 0.50 | 0.50 |
| Component 18 | 45.20 | 36.90 | 36.90 | 36.90 | 38.40 | 38.40 |
| Component 19 | 3.00 | 3.00 | 3.00 | 3.00 | 3.00 | 3.00 |
| Component 20 (c1) | 0.30 | 4.60 | 4.60 | 4.60 | | |
| Component 21 | | | | | 4.60 | 4.60 |
| Component 22 (c1) | | | | | | |
| Component 23 (c1) | | 2.50 | 2.50 | 2.50 | | |
| Component 24 | | | | | 2.50 | 2.50 |
| Component 25 (c1) | | | | | | |
| Component 26 (c1) | | | | | | |
| Component 27 (c1) | | | | | | |
| Component 28 (c2) | | 0.80 | 0.80 | 0.80 | | |
| Component 29 (c2) | | 0.70 | 0.70 | 0.70 | | |
| Component 30 (c2) | | | | | | |
| Component 31 (c2) | | | | | | |
| Component 32 (c2) | | | | | | |
| Component 33 | | | | | | |
| Total | 100.00 | 100.00 | 100.00 | 100.00 | 100.00 | 100.00 |
| Total content of (C) | 0.30 | 8.60 | 8.60 | 8.60 | 0.00 | 0.00 |
| Total content of (B) | 0.00 | 0.00 | 0.00 | 0.00 | 0.00 | 0.00 |
| Material of storage container | PE | PE | PE | PP | PE | PE |

(continued)

|  | Example 28 | Example 29 | Example 30 | Example 31 | Example 32 | Example 33 |
|---|---|---|---|---|---|---|
| (Y) | 0.30 | 0.30 | 0.80 | 0.80 | 0.80 | 0.80 |
| (A)/(B) | - | - | - | - | - | - |
| (A)/(C) | 53.33 | 1.86 | 1.86 | 1.86 | - | - |
| (B)/(C) | 0.00 | 0.00 | 0.00 | 0.00 | - | - |
| (X)/(Y) | 53.3 | 53.3 | 20.0 | 20.0 | 20.0 | 20.0 |
| Dyeability | 3 | 4 | 4 | 4 | 4 | 4 |
| Feeling | 2 | 2 | 2 | 2 | 2 | 2 |
| Storage stability | ○ | ○ | ⊙ | ⊙ | × | × |
| Inner pressure increase | ○ | ○ | ⊙ | ⊙ | ⊙ | ⊙ |

[Table 5]

|  | Example 34 | Example 35 | Example 36 | Example 37 | Example 38 | Example 39 | Example 40 | Example 41 |
|---|---|---|---|---|---|---|---|---|
| Component 1 (A) | 30 (13) | 30 (13) | 30 (13) | 30 (13) | 22 (13) | 30 (13) | 30 (13) | 30 (13) |
| Component 2 |  |  |  |  |  |  |  |  |
| Component 3 |  |  |  |  |  |  |  |  |
| Component 4 | 15.00 | 15.00 | 15.00 | 15.00 | 15.00 | 15.00 | 15.00 | 15.00 |
| Component 5 | 17.00 | 17.00 | 17.00 | 17.00 | 17.00 | 17.00 | 17.00 | 17.00 |
| Component 6 | 1.00 | 1.00 | 1.00 | 1.00 | 1.00 | 1.00 | 1.00 | 1.00 |
| Component 7 | 0.50 | 0.50 | 0.50 | 0.50 | 0.50 | 0.50 | 0.50 | 0.50 |
| Component 8 | 0.50 | 0.50 | 0.50 | 0.50 | 0.50 | 0.50 | 0.50 | 0.50 |
| Component 9 | 0.50 | 0.50 | 0.50 | 0.50 | 0.50 | 0.50 | 0.50 | 0.50 |
| Component 10 (B) |  |  |  |  |  |  |  |  |
| Component 11 (B) | 5.00 | 5.00 | 5.00 | 5.00 | 5.00 | 5.00 | 5.00 | 5.00 |
| Component 12 (B) |  |  |  |  |  |  |  |  |
| Component 13 (B) |  |  |  |  |  |  |  |  |
| Component 14 (B) |  |  |  |  |  |  |  |  |
| Component 15 (B) |  |  |  |  |  |  |  |  |
| Component 16 | 0.50 | 0.50 | 0.50 | 0.50 | 0.50 | 0.50 | 0.50 | 0.50 |

(continued)

| | Example 34 | Example 35 | Example 36 | Example 37 | Example 38 | Example 39 | Example 40 | Example 41 |
|---|---|---|---|---|---|---|---|---|
| Component 17 | 0.50 | 0.50 | 0.50 | 0.50 | 0.50 | 0.50 | 0.50 | 0.50 |
| Component 18 | 18.46 | 16.45 | 23.50 | 22.50 | 2.00 | 17.90 | 17.90 | 17.90 |
| Component 19 | 3.00 | 3.00 | 3.00 | 3.00 | 3.00 | 3.00 | 3.00 | 3.00 |
| Component 20 (c1) | 2.00 | 3.00 | | 1.00 | 20.00 | 4.60 | 4.60 | 4.60 |
| Component 21 | | | | | | | | |
| Component 22 (c1) | | 1.30 | | | 8.50 | | | |
| Component 23 (c1) | | 0.60 | | | | 2.50 | 2.50 | 2.50 |
| Component 24 | | | | | | | | |
| Component 25 (c1) | | | | 0.50 | | | | |
| Component 26 (c1) | | | | | | | | |
| Component 27 (c1) | | | 1.50 | | | | | |
| Component 28 (c2) | | | 1.50 | | | | 0.80 | 0.80 |
| Component 29 (c2) | 0.13 | 0.30 | | | 3.00 | 0.80 | | |
| Component 30 (c2) | 0.01 | | | | | 0.70 | 0.70 | 0.70 |
| Component 31 (c2) | | 0.25 | | | 1.00 | | | |
| Component 32 (c2) | | | | 0.50 | | | | |
| Component 33 | 5.90 | 4.60 | | 2.00 | | | | |
| Total | 100.00 | 100.00 | 100.00 | 100.00 | 100.00 | 100.00 | 100.00 | 100.00 |
| Total content of (C) | 2.14 | 5.45 | 3.00 | 2.00 | 32.50 | 8.60 | 8.60 | 8.60 |
| Total content of (B) | 5.00 | 5.00 | 5.00 | 5.00 | 5.00 | 5.00 | 5.00 | 5.00 |
| Material of storage container | PE | PE | PE | PE | PE | PE | PE | PE |
| (Y) | 0.80 | 0.80 | 0.80 | 0.80 | 0.80 | 0.80 | 0.80 | 0.80 |
| (A)/(B) | 6.00 | 6.00 | 6.00 | 6.00 | 4.40 | 6.00 | 6.00 | 6.00 |
| (A)/(C) | 14.02 | 5.50 | 10.00 | 15.00 | 0.68 | 3.49 | 3.49 | 3.49 |

(continued)

|  | Example 34 | Example 35 | Example 36 | Example 37 | Example 38 | Example 39 | Example 40 | Example 41 |
|---|---|---|---|---|---|---|---|---|
| (B)/(C) | 2.34 | 0.92 | 1.67 | 2.50 | 0.15 | 0.58 | 0.58 | 0.58 |
| (X)/(Y) | 37.5 | 37.5 | 37.5 | 37.5 | 27.5 | 37.5 | 37.5 | 37.5 |
| Dyeability | 5 | 5 | 5 | 5 | 5 | 5 | 5 | 5 |
| Feeling | 5 | 5 | 5 | 5 | 5 | 5 | 5 | 5 |
| Storage stability | ⊙ | ⊙ | ⊙ | ⊙ | ⊙ | ⊙ | ⊙ | ⊙ |
| Inner pressure increase | ⊙ | ⊙ | ⊙ | ⊙ | ⊙ | ⊙ | ⊙ | ⊙ |

[Table 6]

|  | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|
| Component 1 (A comparative) | 30 (11) | 28 (11) | 10 (11) | 53 (11) | 10 (11) | 10(11) | 10 (11) | 10 (11) |
| Component 2 |  |  |  |  |  |  |  |  |
| Component 3 |  |  |  |  |  |  |  |  |
| Component 4 | 15.00 | 15.00 | 15.00 | 15.00 | 15.00 | 15.00 | 15.00 | 15.00 |
| Component 5 | 17.00 | 17.00 | 17.00 | 14.00 | 17.00 | 17.00 | 17.00 | 17.00 |
| Component 6 | 1.00 | 1.00 | 1.00 | 1.00 | 1.00 | 1.00 | 1.00 | 1.00 |
| Component 7 | 0.50 | 0.50 | 0.50 | 0.50 | 0.50 | 0.50 | 0.50 | 0.50 |
| Component 8 | 0.50 | 0.50 | 0.50 | 0.50 | 0.50 | 0.50 | 0.50 | 0.50 |
| Component 9 | 0.50 | 0.50 | 0.50 | 0.50 | 0.50 | 0.50 | 0.50 | 0.50 |
| Component 10 (B) |  | 1.80 | 1.50 | 1.50 |  |  | 5.00 |  |
| Component 11 (B) |  |  |  |  |  |  |  | 5.00 |
| Component 12 (B) |  |  |  |  |  |  |  |  |
| Component 13 (B) |  |  |  |  |  |  |  |  |
| Component 14 (B) |  |  |  |  |  |  |  |  |
| Component 15 (B) |  |  |  |  |  |  |  |  |
| Component 16 | 0.50 | 0.50 | 0.50 | 0.50 | 0.50 | 0.50 | 0.50 | 0.50 |
| Component 17 | 0.50 | 0.50 | 0.50 | 0.50 | 0.50 | 0.50 | 0.50 | 0.50 |
| Component 18 | 22.90 | 23.10 | 41.40 | 1.40 | 51.20 | 42.90 | 37.90 | 37.90 |

(continued)

|  | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|
| Component 19 | 3.00 | 3.00 | 3.00 | 3.00 | 3.00 | 3.00 | 3.00 | 3.00 |
| Component 20 (c1) |  |  |  |  | 0.30 | 4.60 |  |  |
| Component 21 | 4.60 | 4.60 | 4.60 | 4.60 |  |  | 4.60 | 4.60 |
| Component 22 (c1) |  |  |  |  |  |  |  |  |
| Component 23 (c1) |  |  |  |  |  | 2.50 |  |  |
| Component 24 | 2.50 | 2.50 | 2.50 | 2.50 |  |  | 2.50 | 2.50 |
| Component 25 (c1) |  |  |  |  |  |  |  |  |
| Component 26 (c1) |  |  |  |  |  |  |  |  |
| Component 27 (c1) |  |  |  |  |  |  |  |  |
| Component 28 (c2) |  |  |  |  |  | 0.80 |  |  |
| Component 34 | 0.80 | 0.80 | 0.80 | 0.80 |  |  | 0.80 | 0.80 |
| Component 29 (c2) |  |  |  |  |  | 0.70 |  |  |
| Component 35 | 0.70 | 0.70 | 0.70 | 0.70 |  |  | 0.70 | 0.70 |
| Component 30 (c2) |  |  |  |  |  |  |  |  |
| Component 31 (c2) |  |  |  |  |  |  |  |  |
| Component 32 (c2) |  |  |  |  |  |  |  |  |
| Component 33 |  |  |  |  |  |  |  |  |
| Total | 100.00 | 100.00 | 100.00 | 100.00 | 100.00 | 100.00 | 100.00 | 100.00 |
| Total content of (C) | 0.00 | 0.00 | 0.00 | 0.00 | 0.3 | 8.6 | 0.00 | 0.00 |
| Total content of (B) | 0.00 | 1.80 | 1.50 | 1.50 | 0.00 | 0.00 | 5.00 | 5.00 |
| Material of storage container | PVC | PVC | PVC | PVC | PVC | PVC | PVC | PVC |
| (Y) | 0.30 | 0.30 | 0.30 | 0.30 | 0.30 | 0.30 | 0.30 | 0.30 |
| (A)/(B) | - | 15.56 | 6.67 | 35.33 | - | - | 2.00 | 2.00 |
| (A)/(C) | - | - | - | - | 33.33 | 1.16 | - | - |

(continued)

|  | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|
| (B)/(C) | - | - | - | - | 0.00 | 0.00 | - | - |
| (X)/(Y) | 100.0 | 93.3 | 33.3 | 176.7 | 33.3 | 33.3 | 33.3 | 33.3 |
| Dyeability | 2 | 2 | 1 | 2 | 1 | 1 | 1 | 1 |
| Feeling | 1 | 2 | 2 | 2 | 1 | 1 | 3 | 5 |
| Storage stability | × | × | × | × | ○ | ○ | × | × |
| Inner pressure increase | Δ | Δ | Δ | Δ | Δ | Δ | Δ | Δ |

[Table 7]

|  | Compo Ex. 9 | Comp. Ex. 10 | Comp. Ex. 11 | Comp. Ex. 12 | Comp. Ex. 13 | Comp. Ex. 14 | Comp. Ex. 15 | Comp. Ex. 16 |
|---|---|---|---|---|---|---|---|---|
| Component 1 (A comparative) | 10 (11) | 10 (11) | 10 (11) | 10 (11) | 10 (11) | 10 (11) | 10 (11) | 30 (11) |
| Component 2 |  |  |  |  |  |  |  |  |
| Component 3 |  |  |  |  |  |  |  |  |
| Component 4 | 15.00 | 15.00 | 15.00 | 15.00 | 15.00 | 15.00 | 15.00 | 15.00 |
| Component 5 | 17.00 | 17.00 | 17.00 | 17.00 | 17.00 | 17.00 | 17.00 | 17.00 |
| Component 6 | 1.00 | 1.00 | 1.00 | 1.00 | 1.00 | 1.00 | 1.00 | 1.00 |
| Component 7 | 0.50 | 0.50 | 0.50 | 0.50 | 0.50 | 0.50 | 0.50 | 0.50 |
| Component 8 | 0.50 | 0.50 | 0.50 | 0.50 | 0.50 | 0.50 | 0.50 | 0.50 |
| Component 9 | 0.50 | 0.50 | 0.50 | 0.50 | 0.50 | 0.50 | 0.50 | 0.50 |
| Component 10 (B) |  |  |  |  |  |  |  |  |
| Component 11 (B) | 5.00 | 5.00 | 5.00 | 5.00 |  |  |  |  |
| Component 12 (B) |  |  |  |  |  |  |  |  |
| Component 13 (B) |  |  |  |  |  |  |  |  |
| Component 14 (B) |  |  |  |  |  |  |  |  |
| Component 15 (B) |  |  |  |  |  |  |  |  |
| Component 16 | 0.50 | 0.50 | 0.50 | 0.50 | 0.50 | 0.50 | 0.50 | 0.50 |
| Component 17 | 0.50 | 0.50 | 0.50 | 0.50 | 0.50 | 0.50 | 0.50 | 0.50 |
| Component 18 | 46.20 | 37.90 | 37.90 | 37.90 | 51.20 | 42.90 | 42.90 | 22.90 |
| Component 19 | 3.00 | 3.00 | 3.00 | 3.00 | 3.00 | 3.00 | 3.00 | 3.00 |
| Component 20 (c1) | 0.30 | 4.60 | 4.60 | 4.60 | 0.30 | 4.60 | 4.60 |  |
| Component 21 |  |  |  |  |  |  |  | 4.60 |

(continued)

| | Compo Ex. 9 | Comp. Ex. 10 | Comp. Ex. 11 | Comp. Ex. 12 | Comp. Ex. 13 | Comp. Ex. 14 | Comp. Ex. 15 | Comp. Ex. 16 |
|---|---|---|---|---|---|---|---|---|
| Component 22 (c1) ) | | | | | | | | |
| Component 23 (c1) | | 2.50 | 2.50 | 2.50 | | 2.50 | 2.50 | |
| Component 24 | | | | | | | | 2.5 |
| Component 25 (c1) ) | | | | | | | | |
| Component 26 (c1) | | | | | | | | |
| Component 27 (c1) | | | | | | | | |
| Component 28 (c2) | | 0.80 | 0.80 | 0.80 | | 0.80 | 0.80 | |
| Component 34 | | | | | | | | 0.80 |
| Component 29 (c2) | | 0.70 | 0.70 | 0.70 | | 0.07 | 0.70 | |
| Component 35 | | | | | | | | 0.70 |
| Component 30 (c2) | | | | | | | | |
| Component 31 (c2) | | | | | | | | |
| Component 32 (c2) | | | | | | | | |
| Component 33 | | | | | | | | |
| Total | 100.00 | 100.00 | 100.00 | 100.00 | 100.00 | 100.00 | 100.00 | 100.00 |
| Total content of (C) | 0.3 | 8.6 | 8.6 | 8.6 | 0.3 | 8.6 | 8.6 | 0 |
| Total content of (B) | 5.00 | 5.00 | 5.00 | 5.00 | 0.00 | 0.00 | 0.00 | 0.00 |
| Material of storage container | PVC | PVC | PE | PE | PE | PE | PE | PVC |
| (Y) | 0.30 | 0.30 | 0.30 | 0.80 | 0.30 | 0.30 | 0.80 | 2.50 |
| (A)/(B) | 2.00 | 2.00 | 2.00 | 2.00 | - | - | - | - |
| (A)/(C) | 33.33 | 1.16 | 1.16 | 1.16 | 33.33 | 1.16 | 1.16 | - |
| (B)/(C) | 16.67 | 0.58 | 0.58 | 0.58 | 0.00 | 0.00 | 0.00 | - |
| (X)/(Y) | 33.3 | 33.3 | 33.3 | 12.5 | 33.3 | 33.3 | 12.5 | 12.0 |
| Dyeability | 1 | 1 | 1 | 1 | 1 | 1 | 1 | 1 |
| Feeling | 5 | 5 | 5 | 5 | 1 | 1 | 1 | 1 |
| Storage stability | ○ | ○ | ○ | ⊙ | ○ | ○ | ⊙ | × |
| Inner pressure increase | Δ | Δ | ⊙ | ⊙ | ⊙ | ⊙ | ⊙ | × |

Industrial Applicability

In accordance with the invention, the dyeability and bleaching ability of a powder hair dye composition can be enhanced, together with the resulting good hair feeling after application; additionally, the increase of oxygen gas pressure in the storage package can be suppressed, and the storage stability of the powder hair dye composition can be retained in a secure fashion.

**Claims**

1. A storage package obtained by placing a powder hair dye composition for dyeing hair in a storage container with a container wall of a single-layer structure or a multi-layer structure comprising a material selected from polyethylene and polypropylene, wherein the powder hair dye composition contains sodium percarbonate with an active oxygen amount within a range of 12.0 to 15.3 % by mass as a component (A), and additionally contains the sulfate salt of an oxidation dye as a component (C).

2. The storage package according to claim 1, wherein the powder hair dye composition has a content of the component (A) within a range of 20 to 50 % by mass.

3. The storage package according to claim 1 or 2, wherein the powder hair dye composition additionally contains a sequestering agent as a component (B).

4. The storage package according to claim 3, wherein the powder hair dye composition has a content of the component (B) within a range of 2.5 to 10 % by mass.

5. The storage package according to claim 3 or 4, wherein the powder hair dye composition comprises the component (B) being one or more selected from disodium edetate, trisodium edetate, ethylenediaminetetraacetic acid, trisodium ethylenediaminehydroxyethyltriacetate, and diethylenetriaminepentaacetic acid.

6. The storage package according to claims 3 to 5, wherein the powder hair dye composition has a mass ratio (A)/(B) of the content of the component (A) and the content of the component (B) within a range of 2.0 to 16.0.

7. The storage package according to claim 1, wherein the powder hair dye composition comprises the component (C) being one or more selected from the following group (c1) or is a combination of one or more selected from the following group (c1) and one or more selected from the following group (c2):

    (c1) p-phenylenediamine sulfate, toluene-2,5-diamine sulfate, p-aminophenol sulfate, o-aminophenol sulfate, 2,2'-[(4-aminophenyl)imino]bisethanol sulfate, and 1-hydroxyethyl-4,5-diaminopyrazole sulfate; and
    (c2) 5-amino-o-cresol sulfate, m-aminophenol sulfate, 2,6-diaminopyridine sulfate, 2,4-diaminophenoxyethanol sulfate, and p-methylaminophenol sulfate.

8. The storage package according to claim 6 or 7, wherein the powder hair dye composition has a content of the component (C) within a range of 0.25 to 40 % by mass.

9. The storage package according to claims 6 to 8, wherein the powder hair dye composition has a mass ratio (A)/(C) of the content of the component (A) and a content of the component (C) within a range of 0.5 to 160.

10. The storage package according to claims 6 to 9, wherein the powder hair dye composition has a mass ratio (B)/(C) of the content of the component (B) and a content of the component (C) within a range of 0.07 to 35.

11. A storage package according to claim 1, where the thickness of the container wall of the storage container is within a range of 0.5 to 2.0 mm.

12. A storage package according to claim 1 or 11, where the ratio (X)/(Y) under provisions that the numerical figure (X) is in unit "% by mass" of the content of the component (A) in the powder hair dye composition and the numerical figure (Y) is in unit "mm" of the thickness of the container wall of the storage container is within a range of 10 to 80.

**Patentansprüche**

1. Lagerpaket, das erhalten wird, indem ein Pulverhaarfärbegemisch zum Färben von Haar in einem Lagerbehälter mit einer einschichtigen Struktur oder einer mehrschichtigen Struktur vorgehalten wird, die ein Material aufweist, das aus Polyethylen und Polypropylen ausgewählt wird, wobei das Pulverhaarfärbegemisch Natriumpercarbonat mit einem aktiven Sauerstoffanteil innerhalb eines Bereichs von 12,0 bis 15,3 Massenprozent als eine Komponente (A) und zusätzlich das Sulfatsalz eines Oxidationsmittels als eine Komponente (C) enthält.

2.  Lagerpaket nach Anspruch 1, wobei die Komponente (A) zwischen 20 und 50 Massenprozent des Pulverhaarfärbegemisches ausmacht.

3.  Lagerpaket nach Anspruch 1 oder 2, wobei das Pulverhaarfärbegemisch zusätzlich ein Maskierungsmittel als eine Komponente (B) beinhaltet.

4.  Lagerpaket nach Anspruch 3, wobei die Komponente (B) zwischen 2,5 und 10 Massenprozent des Pulverhaarfärbegemisches ausmacht.

5.  Lagerpaket nach Anspruch 3 oder 4, wobei das Pulverhaarfärbegemisch die Komponente (B) aufweist, die aus einem oder mehreren von Dinatrium-EDTA, Trinatrium-EDTA, Ethylendiamintetraessigsäure, Trinatrium-Ethylendiaminhydroxyethyltriacetat und Diethylentriaminpentaessigsäure besteht.

6.  Lagerpaket nach den Ansprüchen 3 bis 5, wobei das Pulverhaarfärbegemisch ein Massenverhältnis (A)/(B) zwischen dem Inhalt der Komponente (A) und dem Inhalt der Komponente (B) innerhalb eines Bereichs zwischen 2,0 und 16,0 aufweist.

7.  Lagerpaket nach Anspruch 1, wobei das Pulverhaarfärbegemisch die Komponente (C) aufweist, die aus einem oder mehreren aus der folgenden Gruppe (c1) ausgewählt wird oder eine Kombination aus einem oder mehreren aus der folgenden Gruppe (c1) und einem oder mehreren aus der folgenden Gruppe (c2) ist:

    (c1) p-Phenylendiaminsulfat, Toluol-2,5-diaminsulfat, p-Aminophenolsulfat, o-Aminophenolsulfat, 2,2'-[(4-Aminophenyl)imino]bisethanolsulfat und 1-Hydroxyethyl-4,5-diaminopyrazolsulfat; und
    (c2) 5-Amino-o-cresolsulfat, m-Aminophenolsulfat, 2,6-Diaminopyridinsulfat, 2,4-Diaminophenoxyethanolsulfat und p-Methylaminophenolsulfat.

8.  Lagerpaket nach Anspruch 6 oder 7, wobei die Komponente (C) zwischen 0,25 und 40 Massenprozent des Pulverhaarfärbegemisches ausmacht.

9.  Lagerpaket nach den Ansprüchen 6 bis 8, wobei das Pulverhaarfärbegemisch ein Massenverhältnis (A)/(C) zwischen dem Inhalt der Komponente (A) und einem Inhalt der Komponente (C) innerhalb eines Bereichs zwischen 0,5 und 160 aufweist.

10. Lagerpaket nach den Ansprüchen 6 bis 9, wobei das Pulverhaarfärbegemisch ein Massenverhältnis (B)/(C) zwischen dem Inhalt der Komponente (B) und einem Inhalt der Komponente (C) innerhalb eines Bereichs zwischen 0,07 und 35 aufweist.

11. Lagerpaket nach Anspruch 1, wobei die Dicke der Behälterwand des Lagerbehälters zwischen 0,5 und 2,0 mm liegt.

12. Lagerpaket nach Anspruch 1 oder 11, wobei das Verhältnis (X)/(Y) der Voraussetzung unterliegt, dass der numerische Wert (X) in der Einheit "Massenprozent" des Inhalts der Komponente (A) in dem Pulverhaarfärbegemisch angegeben ist und der numerische Wert (Y), der in der Einheit "mm" der Dicke der Behälterwand des Lagerbehälters angegeben ist, in einem Bereich von 10 bis 80 liegt.

## Revendications

1.  Emballage de stockage obtenu par le placement d'une composition pulvérulente de teinture capillaire servant à colorer les cheveux dans un récipient de stockage ayant une paroi de récipient à structure monocouche ou à structure multicouche comprenant un matériau choisi parmi le polyéthylène et le polypropylène, dans lequel la composition pulvérulente de teinture capillaire contient du percarbonate de sodium présentant une quantité d'oxygène actif située dans la plage allant de 12,0 % à 15,3 % en poids en tant que composant (A), et contient en outre le sel de sulfate d'un colorant d'oxydation en tant que composant (C).

**2.** Emballage de stockage selon la revendication 1, dans lequel la composition pulvérulente de teinture capillaire présente une teneur en composant (A) située dans la plage allant de 20 % à 50 % en poids.

**3.** Emballage de stockage selon la revendication 1 ou 2, dans lequel la composition pulvérulente de teinture capillaire contient en outre un agent séquestrant en tant que composant (B).

**4.** Emballage de stockage selon la revendication 3, dans lequel la composition pulvérulente de teinture capillaire présente une teneur en composant (B) située dans la plage allant de 2,5 % à 10 % en poids.

**5.** Emballage de stockage selon la revendication 3 ou 4, dans lequel la composition pulvérulente de teinture capillaire comprend, en tant que composant (B), un ou plusieurs composés choisis parmi l'édétate disodique, l'édétate trisodique, l'acide éthylènediaminetétraacétique, l'éthylènediaminehydroxyéthyl-triacétate trisodique et l'acide diéthylènetriaminepentaacétique.

**6.** Emballage de stockage selon les revendications 3 à 5, dans lequel la composition pulvérulente de teinture capillaire présente un rapport pondéral (A)/(B) entre la teneur en composant (A) et la teneur en composant (B) situé dans la plage allant de 2,0 à 16,0.

**7.** Emballage de stockage selon la revendication 1, dans lequel la composition pulvérulente de teinture capillaire comprend, en tant que composant (C), un ou plusieurs composés choisis dans le groupe (c1) suivant ou est une combinaison d'un ou de plusieurs composés choisis dans le groupe (c1) suivant et d'un ou de plusieurs composés choisis dans le groupe (c2) suivant :

(c1) le sulfate de p-phénylènediamine, le sulfate de toluène-2,5-diamine, le sulfate de p-aminophénol, le sulfate d'o-aminophénol, le sulfate de 2,2'-[(4-aminophényl)imino]biséthanol et le sulfate de 1-hydroxyéthyl-4,5-diaminopyrazole ; et
(c2) le sulfate de 5-amino-o-crésol, le sulfate de m-aminophénol, le sulfate de 2,6-diaminopyridine, le sulfate de 2,4-diaminophénoxyéthanol et le sulfate de p-méthylaminophénol.

**8.** Emballage de stockage selon la revendication 6 ou 7, dans lequel la composition pulvérulente de teinture capillaire présente une teneur en composant (C) située dans la plage allant de 0,25 % à 40 % en poids.

**9.** Emballage de stockage selon les revendications 6 à 8, dans lequel la composition pulvérulente de teinture capillaire présente un rapport pondéral (A)/(C) entre la teneur en composant (A) et la teneur en composant (C) situé dans la plage allant de 0,5 à 160.

**10.** Emballage de stockage selon les revendications 6 à 9, dans lequel la composition pulvérulente de teinture capillaire présente un rapport pondéral (B)/(C) entre la teneur en composant (B) et la teneur en composant (C) situé dans la plage allant de 0,07 à 35.

**11.** Emballage de stockage selon la revendication 1, dans lequel l'épaisseur de la paroi de récipient du récipient de stockage est située dans la plage allant de 0,5 mm à 2,0 mm.

**12.** Emballage de stockage selon la revendication 1 ou 11, où le rapport (X)/(Y) est situé dans la plage allant de 10 à 80, à condition que la valeur numérique (X) soit la teneur en composant (A) en « % en poids » dans la composition pulvérulente de teinture capillaire et la valeur numérique (Y) soit l'épaisseur en « mm » de la paroi de récipient du récipient de stockage.